# FASCICULE DE BREVET EUROPEEN

(11) **EP 3 024 380 B1**
(45) Date de publication et mention de la délivrance du brevet: **27.09.2017**
(21) Numéro de dépôt: 14780567.5
(22) Date de dépôt: 25.07.2014
(51) Int. Cl.: A61B 5/00, A61B 5/145, A61B 5/20, A61B 10/00

(54) **DISPOSITIF DE MESURE DE LA PERTE HYDRIQUE**
VORRICHTUNG ZUR MESSUNG VON WASSERVERLUSTEN
DEVICE FOR MEASURING WATER LOSS

(30) Priorité: 25.07.2013 FR 1357327
(43) Date de publication de la demande: 01.06.2016
(73) Titulaire: Commissariat à l'Énergie Atomique et aux Énergies Alternatives, 75015 Paris (FR)
(72) Inventeur: REVOL-CAVALIER, Frédéric, F-38180 Seyssins (FR)
(74) Mandataire: Nony
(86) Numéro de dépôt international: PCT/IB2014/063412
(87) Numéro de publication internationale: WO 2015/011677

(56) Documents cités:
- EP-A1- 0 038 783
- EP-A1- 2 465 418
- WO-A1-2009/025698
- GB-A- 2 116 850
- US-B2- 7 938 030

## Description

La présente invention concerne la mesure de la quantité de sécrétion d'un liquide physiologique.

De nombreux articles font ressortir l'importance de la prise régulière de boissons lors de la pratique d'une activité sportive afin d'éviter tout risque de déshydratation. Cette recommandation est d'autant plus importante que l'effort physique se prolonge et que les conditions climatiques durant l'effort sont contraignantes. Par exemple, la pratique d'une activité physique dans un environnement chaud et humide va entrainer une sudation beaucoup plus importante que cette même activité dans des conditions de température et d'hygrométrie plus faibles.

La perte hydrique due à la transpiration va entrainer une déshydratation chez le sportif qui va ressentir des troubles physiologiques si elle n'est pas compensée. Une déshydratation même légère altère les facultés cognitives fondamentales comme la mémoire à court terme et la mémoire de travail. La prise de décision dans une tache perceptive simple est allongée. On note également une diminution de l'endurance physique des personnes souffrant de déshydratation. Ces pertes de facultés physiques et psychiques sont ressenties dès l'apparition de la déshydratation. Une déshydratation légère (inférieure à 2 % de perte de poids) peut entrainer des pertes de performances non négligeables, de l'ordre de 20 %.

Ceci est préoccupant pour les personnes devant intervenir dans un milieu hostile ou avec des vêtements de protection qui vont augmenter la vitesse de déshydratation. La prise en compte de la perte hydrique est également très importante pour les sportifs voulant se maintenir à leur taux de performance maximal.

Une façon de suivre la déshydratation est la mesure de perte en eau de l'individu. Pour cela, on peut utiliser une balance différentielle qui va permettre de suivre l'individu lors d'un effort physique ou d'une mise en situation de contrainte thermique. La balance va mesurer le poids de l'individu tout au long de l'exercice et donner la perte en eau induite par la sudation. Une fois cette perte ramenée au poids global de l'individu, on peut calculer son niveau de déshydratation. Cette technique est bien adaptée à une étude en laboratoire lors d'exercices spécifiques et contrôlés, mais n'est pas envisageable pour le suivi des personnes au cours d'exercices physiques en extérieur ou d'activités professionnelles courantes.

La demande JP 2010046196 divulgue un système permettant la mesure de la quantité de sudation lors d'un exercice physique ou lors d'une crise cardiaque, composé d'une bandelette d'un matériau absorbant la sueur au fur et à mesure de la sudation. Un indicateur coloré marque la bandelette au fur et à mesure de l'absorption de la sueur par celle-ci. La longueur colorée de la bandelette indique la quantité de sueur collectée et par intégration donne la mesure totale de sudation du corps humain.

Un inconvénient de ce système est que la mesure doit être réalisée sur une partie du corps humain susceptible d'être vue par le porteur du système pour lui permettre de suivre la progression de la quantité absorbée durant la durée d'utilisation du système.

De plus, ce système n'est réellement bien adapté qu'à une mesure relative à un évènement soudain entrainant un pic de sudation sur un temps court. En effet, dans ce cas la bandelette va absorber le volume de liquide généré rapidement et la surface de la bandelette mouillée sera indicative du volume absorbé, si le délai entre l'absorption du liquide dans la bandelette et la lecture est bref. Par contre, si le temps d'attente est long, le liquide aura le temps de s'étaler dans la bandelette par capillarité. Le système décrit dans la demande JP 2010-046196 ne permet donc pas de différencier un volume de sueur absorbé pour un temps donné avec un volume de sueur plus faible absorbé sur un temps plus long. Ainsi, un tel système est relativement bien adapté à une mesure lors d'un évènement ponctuel entrainant une exsudation ponctuelle et une détection rapide après cette exsudation, mais demeure peu adapté à une mesure sur une durée plus longue.

L'article de David McColl, Brian Cartlidge, Patricia Connolly, Real-time monitoring of moisture levels in wound dressings in vitro: An experimental study. International Journal of Surgery (2007) 5, 316e322, relate une méthode de mesure capable de suivre l'évolution du taux d'humidité dans un pansement en temps réel. Le principe utilisé est une mesure d'impédance électrique, permettant la détection de liquides ioniques. Plus le volume de liquide est élevé dans le pansement, plus l'impédance est faible. Plusieurs couples d'électrodes sont répartis à l'interface plaie/pansement et permettent ainsi d'avoir une cartographie du taux d'humidité du pansement.

La publication WO 99/17692 présente un capteur de saturation avec indicateur destiné aux tampons absorbants pour l'hygiène féminine. Ce capteur de saturation est formé par plusieurs détecteurs d'humidité répartis dans la longueur du tampon. Chaque détecteur est composé de deux lamelles et d'un absorbant placé entre les deux. Le détecteur devient conducteur lorsque l'absorbant est mouillé.

Le document GB2116850 divulgue un dispositif de mesure de la quantité de sécrétion d'un liquide physiologique comportant un réservoir et un système de détection de la présence du liquide au sein du réservoir, ce système de détection fournissant une information représentative de la quantité de liquide absorbée par le dispositif.

L'invention vise à proposer un dispositif permettant de connaître relativement précisément la quantité d'un liquide physiologique émise, même sur une longue durée, qui soit pratique et confortable à utiliser et de fonctionnement fiable.

L'invention répond à cet objectif grâce, selon un premier de ses aspects, à un dispositif de mesure de la quantité de sécrétion d'un liquide, notamment physiologique, comportant :
- une pluralité de réservoirs d'accumulation du liquide,
- une pluralité de structures de transfert éventuelles , les réservoirs étant reliés fluidiquement en série depuis une zone de captation dudit liquide,
- un système de détection de la présence du liquide au sein des réservoirs et/ou des structures de transfert, ce système de détection fournissant une information représentative de la quantité de liquide absorbée par le dispositif.

Le liquide peut ne diffuser à partir d'un réservoir vers les suivants qu'après que ce réservoir ait absorbé un volume prédéfini de liquide.

L'invention permet de mesurer relativement précisément la quantité de sécrétion d'un liquide, notamment physiologique au cours du temps, y compris sur une longue durée.

Le dispositif selon l'invention est compatible avec divers modes de mesure de la présence du liquide au sein d'un réservoir et/ou d'une structure de transfert, et notamment avec une mesure électrique, laquelle permet d'informer le porteur du débit et du taux de sel du liquide sécrété. En particulier, l'utilisation de plusieurs électrodes, réparties sur l'ensemble du chemin parcouru par le front de liquide au cours de sa progression, permet une mesure précise et reproductible du débit du liquide absorbé. La présence du liquide au sein d'un réservoir et/ou d'une structure de transfert peut également être détectée par une méthode optique, en particulier la colorimétrie.

Contrairement à des systèmes connus rencontrés dans l'état de la technique, le dispositif selon l'invention permet de s'affranchir du déplacement du front de liquide dans les structures de transfert en fonction du temps, par l'utilisation des réservoirs précités, répartis tout au long du chemin parcouru par le front de liquide. Chaque réservoir s'oppose à la diffusion de liquide vers la structure de transfert suivante, c'est-à-dire agit en quelque sorte comme une « vanne » qui est fermée vis-à-vis de la structure de transfert suivante, tant que le liquide secrété est reçu par le réservoir dans un volume inférieur à un volume prédéfini, de préférence dans un volume inférieur à celui pouvant être absorbé par celui-ci. C'est seulement une fois l'absorbant du réservoir saturé ou presque que le liquide supplémentaire secrété va pouvoir franchir ce réservoir et être drainé par la structure de transfert suivante vers le réservoir suivant.

### Réservoir

Le nombre de réservoirs peut être compris entre 2 et 50, mais plus usuellement de 1 à 10.

De préférence, le liquide ne diffuse à partir d'un réservoir vers le suivant qu'après que ce réservoir ait absorbé un volume prédéfini de liquide. Ce volume dépend de paramètres de fabrication du réservoir tels que le matériau utilisé, la géométrie du réservoir et le volume initial du matériau constituant le réservoir.

Les réservoirs peuvent être formés par tout matériau absorbant, et comporter un polymère superabsorbant. Par « matériau » on désigne un matériau unique ou un ensemble de matériaux différents qui sont associés ensemble, auquel cas le matériau est par exemple un matériau composite comportant un matériau superabsorbant mélangé à ou disposé entre un ou plusieurs autres matériaux, servant de support au matériau superabsorbant.

La capacité d'absorption d'eau d'un réservoir est supérieure à celle d'une structure de transfert. Un réservoir peut absorber par exemple plus de 500 g/m² d'eau jusqu'à saturation, mieux plus de 800 g/m², cette valeur pouvant atteindre voire dépasser 5 000 g/m².

Le dispositif selon l'invention peut comporter au moins deux réservoirs disjoints, de préférence identiques, reliés par des structures de transfert.

Les réservoirs peuvent être chacun en contact permanent avec une structure de transfert amont et une structure de transfert aval. En variante, comme détaillé ci-après, le contact avec la structure de transfert aval ne s'opère qu'après que le réservoir ait reçu le liquide. Le réservoir situé le plus en amont peut n'être en contact qu'avec une structure de transfert aval, tandis que le réservoir situé le plus en aval peut n'être en contact qu'avec une structure de transfert amont.

Les réservoirs comportent, de préférence, un matériau capable de gonfler en présence du liquide, de manière à subir une expansion ; le dispositif peut comporter alors une structure de transfert aval, espacée de chaque réservoir correspondant avant expansion de celui-ci, le réservoir venant après son expansion contacter cette structure de transfert aval. Le réservoir peut également comporter un matériau poreux, par exemple un matériau fritté qui, en absorbant un liquide par capillarité, permet à ce dernier de contacter la structure de transfert aval.

Ainsi, le matériau choisi permet une isolation fluidique entre un réservoir et la structure de transfert suivante tant que le réservoir n'a pas absorbé la quantité de liquide nécessaire pour contacter la structure de transfert aval, notamment la quantité de liquide conduisant au gonflement nécessaire pour contacter la structure de transfert aval.

D'une façon générale, le réservoir peut comporter initialement un espace qui résulte en une isolation fluidique entre le réservoir et la structure de transfert et/ou le réservoir suivant. Sous l'effet de l'absorption de liquide, cet espace se comble progressivement, jusqu'à ce qu'un pont fluidique s'établisse entre le réservoir et la structure de transfert et/ou le réservoir aval.

Par « *matériau capable de gonfler* » il faut comprendre un matériau qui subit une augmentation de son volume en présence d'eau, par exemple d'au moins 10 %, mieux 200 %, voire au moins 500 % ou 3 000 %. Il peut s'agir d'un matériau composé d'un polymère superabsorbant ou comportant un tel polymère.

Le matériau capable de gonfler peut ainsi être un hydrocolloïde qui gonfle au contact des liquides, sous forme de poudre, de granulés ou de fibres, un matériau alvéolaire hydrogonflant comme par exemple une mousse de polyuréthane hydrophile ou un gel hydrophile comme par exemple un gel de polyuréthane hydrophile. Comme exemples d'hydrocolloïdes on peut citer les dérivés de cellulose comme par exemple les sels de métal alcalin de la carboxyméthylcellulose, les alginates ou les polymères superabsorbants (SAP).

A titre de polymère superabsorbant (SAP), on peut utiliser un ou plusieurs polymères capables par exemple d'absorber de 10 à 50 fois leur poids d'eau (représentatif d'un liquide physiologique), et de ne pas relarguer l'eau en cas de pression modérée sur ceux-ci. La capacité d'absorption d'eau est due à l'interaction puissante entre les molécules d'eau et des groupements hydrophiles du polymère, notamment capables d'établir une liaison hydrogène. Les polymères superabsorbants peuvent être choisis notamment parmi les copolymères greffés à base d'amidon, les dérivés réticulés de carboxyméthylcellulose et les polyacrylates hydrophiles modifiés. Plus particulièrement, il peut s'agir de copolymères d'amidon et d'acrylonitrile hydrolysés, de copolymères d'amidon et d'acide acrylique neutralisés, de copolymères saponifiés, d'esters d'acide acrylique et vinylacétate, de copolymères d'acrylonitrile hydrolysés, de copolymères d'acrylamide, d'alcools polyvinyliques réticulés modifiés, de sels de polyacrylates réticulés, d'acide polyacrylique neutralisé et réticulé, de cellulose carboxylée ou d'un de leurs mélanges.

Généralement, ce matériau est mis en oeuvre à l'état pulvérulent et ce n'est qu'au contact d'un fluide, en l'occurrence un liquide physiologique, qu'il va former un gel doté de propriétés de rétention.

A titre de matériaux superabsorbants préférés, on peut plus particulièrement citer les polyacrylates de sodium comme par exemple ceux commercialisés sous le nom de Favor^{®}-Pac 230 ou Luquasorb^{®} 1161.

A titre de matériau alvéolaire hydrogonflant, on peut également utiliser une mousse de polyuréthane hydrophile (PU) à l'image par exemple de celle commercialisée sous la dénomination MCF.03 par la société Advanced Médical Solution (AMS).

Comme matériau composite, on peut citer des matériaux constitués des hydrocolloïdes cités précédemment incorporés dans une matrice à base de polymères, comme par exemple les compositions adhérentes hydrocolloïdes utilisées dans le domaine des pansements ou de la stomie, ou un matériau textile comme par exemple les non tissés absorbants incorporant des particules de SAP, couramment utilisés dans le domaine de l'hygiène.

On préfère l'utilisation de non tissés obtenus par la méthode de fabrication par voie sèche connue sous le nom de voie aérodynamique ou « airlaid », qui contiennent des particules de SAP et en particulier entre 20 à 60 % en poids de SAP par rapport au poids total du non tissé. De tels non tissés sont par exemple commercialisé par la société EAM Corporation sous la référence Novathin^{®}.

Selon un mode préféré de mise en oeuvre de la présente invention, on utilise pour réaliser les réservoirs des polymères SAP de type airlaid précédemment évoqués.

Les réservoirs peuvent être superposés en vue de dessus. Cela peut faciliter l'obtention d'un dispositif compact. En variante, les réservoirs sont non superposés en vue de dessus.

Les réservoirs peuvent être disposés chacun au moins partiellement au sein d'un logement réalisé dans une couche d'un matériau hydrophobe.

Au moins une structure de transfert peut définir une zone de transfert aval d'un réservoir et une zone de transfert amont du réservoir suivant. Le dispositif peut comporter une structure formant entretoise, maintenant espacées les zones de transfert amont et aval associées à un même réservoir.

La structure formant entretoise est hydrophobe et non absorbante, de façon à isoler sur le plan fluidique les zones de transfert amont et aval, là où aucune expansion des réservoirs n'a eu lieu. Elle doit aussi éviter la migration du liquide et l'empêcher de passer d'un réservoir à l'autre autrement que par la structure de transfert correspondante. De préférence, elle est souple pour pouvoir se conformer aux contours anatomiques.

La structure formant entretoise est par exemple un matériau alvéolaire à cellules fermées sous la forme d'une couche d'une mousse d'un matériau thermoplastique, notamment une polyoléfine, par exemple du polyéthylène, ou un assemblage de plusieurs couches de ce type. Elle peut aussi se présenter sous la forme d'un film, d'un matériau textile ou d'une couche à base d'adhésifs, de polymères ou d'élastomères, hydrophobes et non absorbants, tels le polyuréthane, le polydiméthylsiloxane et ses déclinaisons (désignés généralement sous le terme générique de « silicone »), les polyoléfines (comme par exemple le polyéthylène) le polyphénylène éther ou des formulations à base de polymères séquencés, par exemple du type( styrène - oléfine - styrène) ou (styrène - oléfine) associés, à un plastifiant.

L'épaisseur de la structure formant entretoise est par exemple comprise entre 0,5 mm et 20 mm, étant de préférence supérieure à celle des réservoirs.

La structure formant entretoise est de préférence ajourée, et l'expansion d'un réservoir a alors lieu au moins partiellement dans un ajour de la structure formant entretoise. Les ajours peuvent avoir toute forme. Les ajours peuvent être formés par découpe dans une feuille, en ligne au cours de la fabrication du dispositif, ou être découpés lors d'une opération préalable. Les ajours peuvent avoir tous le même contour ou non, avec par exemple une répartition des ajours qui est régulière ou non. Les ajours peuvent être de contour circulaire ou polygonal, notamment polygonal régulier. Les ajours peuvent être de section constante sur toute l'épaisseur de la structure formant entretoise, ou en variante avoir une section qui varie, notamment décroît en direction de la zone de transfert amont, de façon à favoriser l'expansion du réservoir en direction de la zone de transfert aval plutôt que dans la direction inverse. Pour obtenir une section qui décroît, on peut utiliser par exemple plusieurs feuilles dans lesquelles les ajours sont découpés avec des tailles décroissantes, puis procéder à l'assemblage desdites feuilles. On peut encore découper les ajours directement avec la forme souhaitée, par exemple en utilisant un laser.

La structure formant entretoise peut être réalisée à l'aide un matériau rigide, tel un plastique, par exemple du Plexiglas, ou un matériau souple, comme une mousse hydrophobe à cellules fermées en polyéthylène, de marque Alveolit, référence TEE. 1000.8, d'épaisseur comprise entre 5 et 10 mm par exemple.

### Structures de transfert

Les structures de transfert servent préférentiellement à la diffusion du liquide de la zone de captation du liquide au premier réservoir, puis de ce réservoir aux suivants. Cette diffusion peut être une diffusion latérale. Par « diffusion latérale » il faut comprendre une diffusion dans une direction du dispositif sensiblement parallèle à la surface sur laquelle est placé le dispositif lors de son utilisation. La diffusion peut aussi avoir lieu dans le sens de l'épaisseur de la structure de transfert, lorsque celle-ci s'interpose entre deux réservoirs superposés l'un à l'autre.

Les structures de transfert peuvent comporter tout matériau drainant, dans lequel le liquide peut facilement diffuser par capillarité.

Les structures de transfert peuvent ainsi comporter ou être constituées par des bandes drainantes, comportant des fibres absorbantes, notamment d'origine végétale, telles que des fibres de cellulose ou de viscose ou leurs dérivés. Les structures de transfert peuvent se présenter sou forme de tricots, tissés, ou non tissés, obtenus par voie sèche ou humide, ou de papier. Les fibres absorbantes peuvent être associées à des fibres non absorbantes, par exemple des fibres de polyester ou de polyoléfine. Il peut par exemple s'agir d'un matériau non tissé JETTEX^{©} 1205c de la société ORSA.

En présence d'une structure formant entretoise, maintenant espacées les zones de transfert amont et aval associées à un même réservoir, les structures de transfert peuvent comporter une portion amont qui reçoit le fluide d'un réservoir et une portion aval qui transmet le fluide au réservoir suivant et une portion intermédiaire qui relie les portions amont et aval et traverse la structure formant entretoise. Le dispositif peut comporter une électrode sur la portion intermédiaire.

La capacité d'absorption de liquide d'une structure de transfert reliant deux réservoirs successifs est de préférence inférieure à la capacité d'absorption de liquide de chacun de ces réservoirs.

Les structures de transfert n'ont pas la même composition que les réservoirs, et peuvent être dépourvues de matériaux superabsorbants.

L'épaisseur d'une structure de transfert peut être comprise entre 50 et 250 µm.

Dans une variante, le dispositif est sans bandes drainantes, donc sans structures de transfert, les réservoirs étant constitués d'un matériau gonflant et étant superposés, avec par exemple des électrodes qui détectent la présence de liquide à l'interface entre deux réservoirs ou au niveau d'un réservoir. Dans ce cas, les réservoirs sont connectés fluidiquement l'un à l'autre après leur remplissage.

On peut également avoir une variante où certains réservoirs sont reliés entre eux par une ou plusieurs structures de transfert et d'autres le sont directement, sans passer par une structure de transfert.

### Electrodes

Les électrodes permettant de détecter la présence de liquide au niveau d'un réservoir ou d'une structure de transfert peuvent être réalisées de diverses façons.

La mesure électrique réalisée à l'aide des électrodes peut être une mesure de résistance électrique du milieu ; on mesure alors la variation de la conductivité du réservoir ou de la structure de transfert, en présence ou non du liquide.

La mesure électrique peut être une mesure de tension électrique aux bornes d'une résistance électrique reliant les différentes électrodes du dispositif.

La mesure électrique peut être une mesure électrochimique par application d'une tension entre deux électrodes du circuit et hydrolyse du liquide s'il est présent entre ces deux électrodes.

Une mesure de capacité électrique entre deux électrodes peut également être réalisée, la capacité électrique variant entre l'état sec et l'état humide.

L'intérêt d'utiliser une mesure électrique pour détecter la présence de liquide au niveau des réservoirs et/ou des structures de transfert est que l'on peut également coupler à cette mesure de débit une mesure de conductivité du liquide absorbé.

En effet, il est possible en appliquant une tension électrique prédéfinie entre deux électrodes voisines ou en vis-à-vis de mesurer la conductivité du liquide. La conductivité du liquide secrété permet de renseigner sur les concentrations ioniques de celui-ci et peut permettre, dans le cas où le liquide recueilli est la sueur, d'adapter la solution à boire ou à injecter pour remplacer le liquide physiologique perdu.

Par exemple, pour la perte sudorale, il est important de connaître la concentration en sel perdue lors d'un exercice physique pour pouvoir la compenser dans la boisson énergétique à prendre en fin d'exercice.

Les électrodes peuvent être filaires ou réalisées par sérigraphie, gravure ou métallisation. Les électrodes peuvent être reliées les unes aux autres par un isolant électrique qui assure leur fixation, le cas échéant.

Le système de détection peut être relié à un système de mesure qui détermine par exemple l'impédance entre deux électrodes consécutives, pour au moins plusieurs des couples d'électrodes pouvant être générés. Par exemple, le système de mesure injecte une tension alternative entre deux électrodes et mesure l'amplitude du courant circulant dans ces électrodes, ce courant étant par exemple déterminé par mesure de la tension de crête aux bornes d'une résistance parcourue par ce courant. Le système de détection peut être relié au système de mesure par une ou plusieurs connexions, de telle sorte que le système de mesure est aisément déconnectable du système de détection.

La tension alternative injectée est par exemple un signal basse fréquence, de fréquence inférieure à 25 kHz. Le signal est par exemple de forme d'onde en créneaux. L'amplitude de la tension injectée est par exemple inférieure ou égale à 5 V.

Le dispositif peut comporter un moyen de fixation sur la peau, notamment un bracelet. Le dispositif peut être disposé de façon à capter la sueur au niveau du front, des aisselles, des avant-bras, du buste.

L'invention a encore pour objet, selon un autre de ses aspects, un procédé de mesure de la quantité d'un liquide physiologique sécrété par une zone de peau, à l'aide d'un dispositif selon l'invention, comportant :
- la détection de la présence du liquide au niveau des structures de transfert et/ou des réservoirs, et
- la fourniture d'une information représentative de la quantité de liquide sécrétée, à partir desdites mesures.

Le procédé peut également comporter une mesure de conductivité permettant de connaître la perte en sel par sudation.

Le procédé peut faire intervenir un outil informatique permettant de délivrer une recommandation sur une prise de boisson et/ou de sel permettant de compenser les pertes hydriques et/ou de sel par sudation, telles que déterminées à l'aide du dispositif.

La mesure peut être transmise à distance. Le dispositif de mesure peut être intégré à une combinaison ou à un casque, le cas échéant.

Le dispositif s'applique tout particulièrement à un liquide physiologique, mais également à tout autre liquide, produit par une source, dès lors que l'on souhaite déterminer la quantité de liquide sécrétée par cette source.

L'invention pourra être mieux comprise à la lecture de la description détaillée qui va suivre, d'exemples de mise en oeuvre non limitatifs de celle-ci, et à l'examen du dessin annexé, sur lequel :
- la figure 1 représente en vue de dessus, de façon schématique et partielle, un exemple de réalisation du dispositif selon l'invention,
- la figure 2 représente, de façon schématique et partielle, une variante de dispositif selon l'invention, en coupe dans le sens de l'épaisseur,
- la figure 3 est une vue analogue à la figure 2, d'une variante du dispositif selon l'invention,
- la figure 4 représente le dispositif de la figure 3, après gonflement du premier réservoir,
- la figure 5 est une vue analogue à la figure 2, d'une variante du dispositif,
- la figure 6 représente une variante de dispositif, en place sur l'avant-bras,
- la figure 7 représente isolément l'embase du dispositif de la figure 6, en vue de dessus,
- la figure 8 est une vue en coupe représentant l'embase de la figure 7 et un premier étage de support, et
- la figure 9 est une vue analogue à la figure 8, avec un étage de support supplémentaire, porté par l'embase.

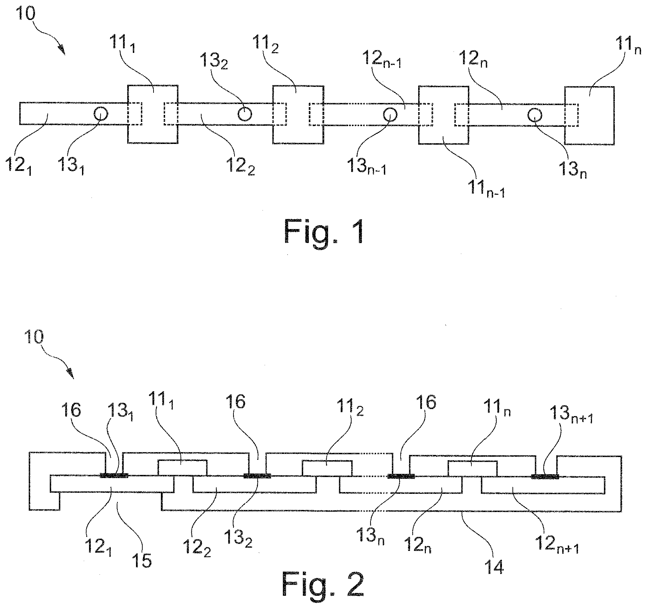

Le dispositif 10 représenté à la figure 1 comporte, conformément à l'invention, une pluralité de réservoirs 11 reliés fluidiquement en série par l'intermédiaire de structures de transfert 12₁ à 12ₙ, constituées ici par des bandes drainantes comportant des fibres absorbantes.

La première bande drainante 12₁ est reliée fluidiquement à la zone de captation du liquide qui est sécrété ou définit cette zone de captation. La première bande drainante peut venir au contact de la peau ou au contact d'une couche d'un matériau absorbant, lui-même en contact avec la peau.

Une pluralité d'électrodes 13₁, 13ₙ est disposée sur les structures de transfert 12₁, ..., 12ₙ de façon à pouvoir détecter la présence du liquide au sein de celles-ci, de préférence par une mesure de la conductivité électrique. La disposition des électrodes peut être variable. La mesure s'effectue par exemple entre deux électrodes 13ᵢ ou entre chacune de ces électrodes et une électrode de référence, non représentée sur le schéma. On peut avoir une électrode de référence associée à chaque électrode de mesure, l'électrode de mesure et l'électrode de référence étant par exemple en face l'une de l'autre ; toutes les électrodes de référence sont par exemple reliées électriquement à la masse.

Au cours de l'utilisation du dispositif, le liquide collecté au niveau de la zone de captation diffuse par capillarité dans la première structure de transfert 12₁ et gagne ainsi le premier réservoir 11₁.

Le liquide ne diffuse pas immédiatement, dès son arrivée dans un réservoir 11ᵢ, dans la structure de transfert suivante 12ᵢ₊₁.

La présence du réservoir 11ᵢ retarde en effet la diffusion du front de liquide et la structure de transfert 12ᵢ₊₁ ne commence à diffuser le liquide vers le réservoir suivant 11ᵢ₊₁ qu'à partir du moment où le réservoir 11ᵢ a absorbé un volume de liquide prédéfini et devient saturé en liquide.

Lorsque le liquide est absorbé par une structure de transfert 12ᵢ, la conductivité du matériau qui la constitue change et la variation de la résistance électrique est mesurée par les électrodes correspondantes, par exemple par une électrode 13ᵢ et une électrode de référence disposées de part et d'autre de la structure de transfert 12ᵢ. Cette modification de la conductivité renseigne sur la progression du liquide et une information correspondante peut être transmise à l'utilisateur, qui est ainsi alerté sur la quantité de liquide secrété.

L'utilisateur peut être, d'une façon générale, informé de diverses façons. Par exemple, l'utilisateur reçoit un message visuel affiché sur un écran ou un message sonore qui l'informe sur le taux de perte hydrique et/ou de perte de sel. Une préconisation sur une quantité de boisson à prendre pour compenser les pertes peut être émise. Le système de détection qui comporte les différentes électrodes 13ᵢ peut être relié à un système de mesure local, qui communique avec un dispositif distant par une liaison filaire ou sans fil. Par exemple, le dispositif distant est un téléphone portable de type smartphone, une montre communiquante, un boîtier porté par l'utilisateur ou un panneau de contrôle installé sur un véhicule ou dans un bâtiment, et la liaison avec le dispositif de mesure s'effectue par exemple par un protocole de transmission sans fil tel que Bluetooth, Wifi, ou autre. Le dispositif selon l'invention peut également être intégré à une montre, dont la face interne en regard de la peau reçoit un module amovible définissant la zone de captation, comportant les réservoirs et les structures de transfert, ou à un boîtier tel qu'un cardiofréquencemètre et/ou podomètre porté par une ceinture à la taille.

Il peut être intéressant que la surface de contact entre une structure de transfert et un réservoir adjacent soit relativement faible, par exemple moins de 50 % de la surface du réservoir adjacent, afin de réduire le risque de diffusion de liquide dans la structure de transfert suivante avant que le réservoir ne soit complètement saturé de liquide. De préférence, deux structures de transfert consécutives sont disjointes, comme illustré. Il en est de même des réservoirs.

La figure 2 représente une variante de réalisation du dispositif 10 de la figure 1, dans laquelle une couche 14 d'un matériau imperméable permet d'isoler les structures de transfert 12ᵢ de la surface de la peau, sauf dans une ouverture 15 qui définit la zone de captation du liquide. Le matériau imperméable de la couche 14 peut être perméable à la vapeur d'eau et à l'air.

Les structures de transfert 12ᵢ et les réservoirs 11ᵢ peuvent être recouverts par la couche 14 de matériau imperméable, à l'exception par exemple de zones 16 qui permettent d'accéder aux électrodes 13ᵢ disposées au contact des structures de transfert 12ᵢ.

Une face externe de la couche 14 peut être recouverte localement d'une colle ou d'un matériau permettant de fixer le dispositif 10 sur la personne.

La première structure de transfert 12ᵢ étant en contact avec la zone de sécrétion à travers l'ouverture 15, le liquide secrété est amené sur le système de mesure par cette première structure de transfert 12₁. Le reste du système de mesure est isolé du milieu extérieur par la couche 14 de matériau imperméable à l'eau.

A fur et à mesure du drainage du liquide physiologique par le dispositif 10, le liquide va progresser et va être absorbé successivement par les réservoirs 11ᵢ répartis sur la longueur du dispositif.

La modification de la conductivité des matériaux utilisés, lorsqu'ils sont secs ou imbibés par le liquide physiologique, peut être détectée par une mesure électrique.

Le suivi des mesures électriques sur les différentes électrodes 13ᵢ réparties tout au long du dispositif 10 permet de connaître la quantité de liquide physiologique exsudé. On peut également estimer le début de la sudation, si la première électrode 13₁ est située très près de la zone de contact avec la peau.

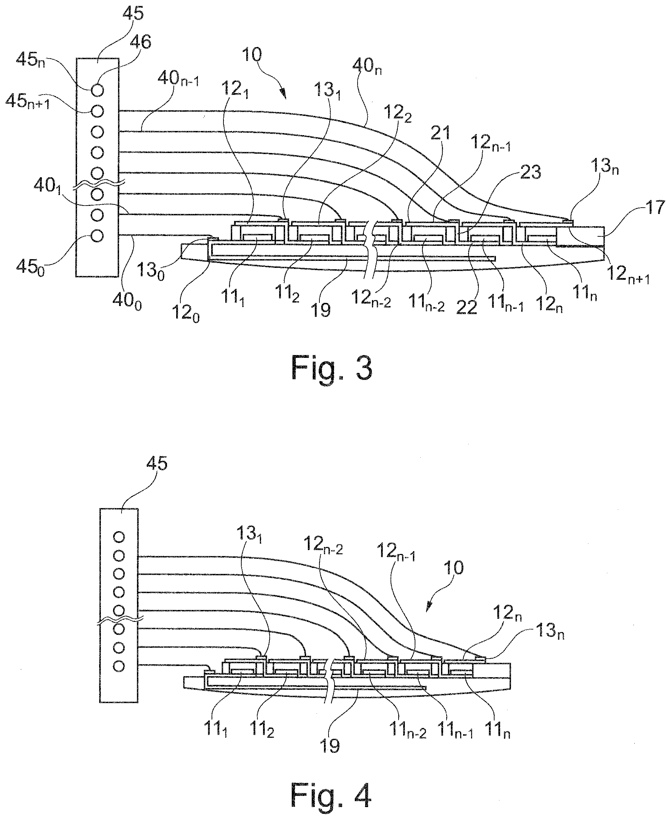

Dans l'exemple des figures 3 et 4, les réservoirs 11ᵢ comportent un matériau pouvant subir une expansion en gonflant au contact du liquide, lors de l'absorption de celui-ci. Les réservoirs 11ᵢ peuvent ainsi chacun subir une expansion, notamment dans le sens de leur épaisseur, entre des zones de transfert amont et aval.

Le liquide gagne un réservoir donné 11ᵢ par la zone de transfert amont et le quitte par la zone de transfert aval.

Chaque réservoir 11ᵢ est en contact permanent avec une structure de transfert amont correspondante 12ᵢ₋₁ et peut venir, au terme de son expansion, au contact de la structure de transfert aval correspondante 12ᵢ.

Dans l'exemple considéré, les structures de transfert amont et aval sont définies par des bandes drainantes respectives qui traversent chacune une structure formant entretoise 17. Plus particulièrement, chaque bande drainante définit la zone de transfert aval du réservoir précédent et la zone de transfert amont du réservoir suivant.

Chaque bande drainante possède ainsi une portion amont 21 qui définit la zone de transfert aval et une portion aval 22 qui définit la zone de transfert amont, et une portion intermédiaire 23 qui relie les deux et traverse la structure formant entretoise 17.

La structure formant entretoise 17 peut être formée d'au moins une couche d'un matériau hydrophobe et/ou imperméable à l'eau, qui est ajourée pour définir des logements recevant les réservoirs respectifs 11ᵢ.

Une électrode 13ᵢ peut être fixée sur chaque bande drainante, par exemple sur la portion intermédiaire 23 avant traversée de la structure formant entretoise 17, comme illustré.

Dans l'exemple des figures 3 et 4, le premier réservoir 11ᵢ est au contact d'une bande drainante 12₀ qui est repliée sur elle-même pour définir la zone de captation du liquide.

La portion 19 qui définit cette zone de captation s'étend par exemple sous plusieurs réservoirs, comme illustré.

Une électrode 13₀ peut être fixée sur cette bande drainante, par exemple en amont du premier réservoir 11₁, après la portion de collecte 19. Une dernière électrode 13ₙ peut être fixée sur la dernière bande drainante 12ₙ₊₁, qui n'est reliée de façon permanente à aucun réservoir, mais qui est contactée par le dernier réservoir 11ₙ au terme de son expansion.

Lors de l'utilisation du dispositif 10, le liquide diffuse grâce à la première bande drainante 12₀ depuis la portion de collecte 19 vers la zone de transfert amont associée au premier réservoir 11₁. Ce dernier se charge de liquide et gonfle, comme illustré à la figure 4, jusqu'à contacter, au terme de l'absorption d'un volume de liquide prédéfini, la zone de transfert aval correspondante, définie par la structure de transfert 12₁ qui relie fluidiquement les premier 11₁ et deuxième 11₂ réservoirs. Cette structure de transfert 12₁ assure la diffusion du liquide depuis la zone de transfert aval associée au premier réservoir 11₁ vers la zone de transfert amont associée au deuxième réservoir 11₂. Il en va de même au fur et à mesure de la progression du liquide vers le dernier réservoir 11ₙ, puis au sein de la dernière structure de transfert 12ₙ₊₁ qui amène le liquide jusqu'à la dernière électrode 13ₙ de détection.

Dans l'exemple des figures 3 et 4, on a représenté des conducteurs électriques 40₀, 40₁, ..., 40ₙ₋₁, 40ₙ qui relient respectivement les électrodes 13₀, ..., 13ₙ à un système de mesure 45. Celui-ci comporte par exemple un afficheur 46 de type bar-graph, dont chaque segment 46ᵢ correspond à une électrode respective 13ᵢ et s'allume lorsque le liquide a atteint cette électrode.

Bien entendu, l'affichage peut être réalisé différemment.

Les conducteurs électriques 40ᵢ peuvent être reliés à un dispositif de mesure proche des réservoirs et structures de transfert, le cas échéant. Ce dispositif de mesure est par exemple réalisé sous forme d'un module de petite taille qui est intégré dans l'une des couches du dispositif 10 et qui peut transmettre à distance une information représentative de la progression du liquide au sein du dispositif 10.

Dans l'exemple des figures 3 et 4, les réservoirs 11ᵢ sont disposés selon un même plan, en se succédant dans une direction.

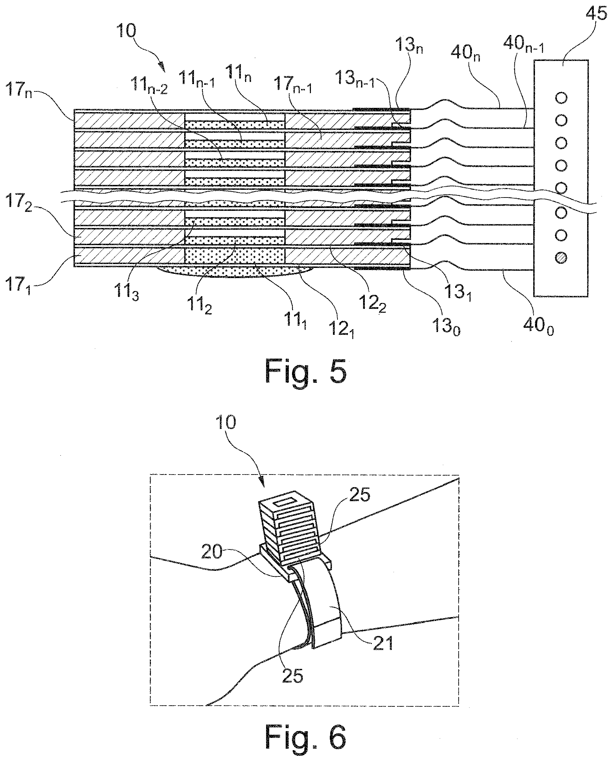

La figure 5 représente une autre réalisation dans laquelle les réservoirs 11ᵢ sont superposés lorsque le dispositif 10 est observé en vue de dessus.

Le dispositif 10 comporte dans cet exemple une pluralité de structures intermédiaires formant entretoise 17ᵢ, qui permettent d'espacer verticalement les réservoirs 11ᵢ entre eux.

Dans l'exemple illustré, chaque réservoir 11ᵢ est porté par une structure de transfert 12ᵢ qui définit la zone de transfert aval du réservoir précédent 11ᵢ₋₁ et la zone de transfert amont du réservoir 11ᵢ correspondant.

Ces structures de transfert 12ᵢ sont de préférence constituées, comme illustré, de bandes drainantes qui sont plus grandes, en vue de dessus, que les réservoirs 11ᵢ correspondants, ce qui permet d'y positionner sur le côté des électrodes de détection correspondantes 13ᵢ comme illustré, à distance des réservoirs.

Sur la figure 5, on a représenté le premier réservoir 11₁, après expansion sous l'effet de son gonflement en présence de liquide. On peut voir que ce premier réservoir contacte alors la bande drainante 12₂ qui le recouvre immédiatement.

La configuration empilée de la figure 5 convient tout particulièrement à la réalisation d'un dispositif 10 tel que représenté aux figures 6 à 9.

Le dispositif 10 représenté sur ces figures 6 à 9 comporte une embase 20 qui peut recevoir un bracelet 21, comme illustré, pour permettre une fixation sur l'avant-bras par exemple.

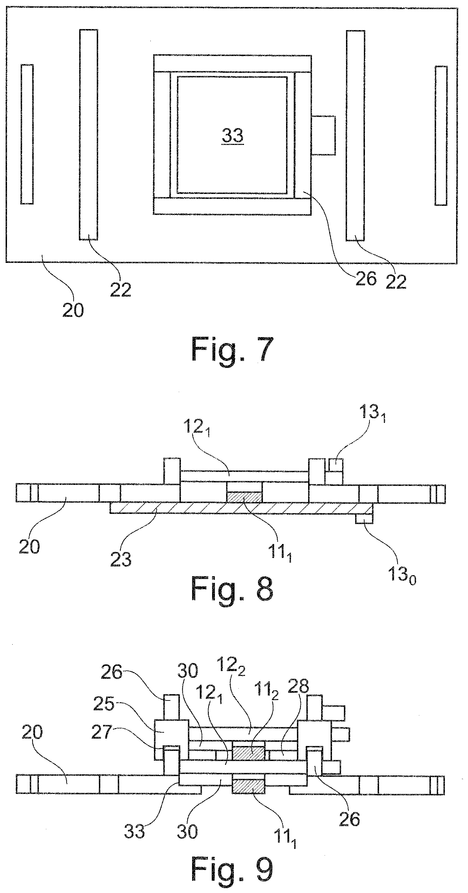

L'embase 20 comporte à cet effet des ouvertures 22, représentées plus particulièrement à la figure 7, qui permettent l'accrochage du bracelet 21.

L'embase 20 comporte un support en matière thermoplastique, sur lequel peut être fixée une couche 23 d'un matériau de collecte, laquelle définit la zone de transfert amont du premier réservoir 11₁. Ce matériau de collecte peut être formé par tout matériau absorbant.

L'embase 20 reçoit des cadres de support 25 superposés, qui se positionnent sur l'embase 20 grâce à des reliefs coopérants.

Par exemple, chaque cadre 25 présente en partie inférieure une gorge 27 et en partie supérieure une nervure 26 agencée pour s'engager dans la gorge 27 du cadre qui est posé dessus. L'embase 20 peut être réalisée avec une nervure 26 identique à celle des cadres 25.

L'embase 20 présente un logement 33 qui reçoit un substrat hydrophobe 30 porteur du premier réservoir 11₁. Le substrat 30 est par exemple, comme illustré, ajouré en son centre et le premier réservoir est disposé dans l'ajour, de façon à ce que sa face inférieure vienne au contact de la couche de collecte 23, comme illustré à la figure 8. Il peut s'agir d'un matériau similaire à l'entretoise 17.

Chaque cadre 25 présente un rebord 28 qui supporte un ensemble constitué du substrat hydrophobe 30, de la structure de transfert 12ᵢ et du réservoir 11ᵢ.

Le positionnement des cadres 25 fait en sorte que la face inférieure d'un réservoir 11ᵢ porté par un cadre contacte la structure de transfert 12ᵢ₋₁ du cadre sous-jacent.

Le positionnement et l'épaisseur du rebord 28, et l'épaisseur du substrat 30, sont tels que la face supérieure de chaque réservoir 11ᵢ ne contacte pas la face inférieure de la structure de transfert 12ᵢ qui lui est immédiatement superposée, tant que le réservoir 11ᵢ n'a pas gonflé suite à l'absorption du liquide.

Des électrodes 13ᵢ sont prévues à chaque étage du dispositif 10, au contact de la structure de transfert 12ᵢ correspondante, pour détecter l'arrivée du liquide dans cette structure de transfert.

Le système de mesure, non représenté, relié aux électrodes peut être porté par l'embase 20 et transmettre le cas échéant l'information concernant l'état des mesures à distance.

Bien entendu, l'invention n'est pas limitée aux exemples de réalisation qui viennent d'être décrits. On peut apporter de nombreuses modifications sans sortir du cadre de la présente invention. Par exemple, les réservoirs peuvent être réalisés avec d'autres géométries, de même que les structures de transfert.

Dans une variante, le dispositif de la figure 5 est réalisé sans les structures de transfert 12i. Dans ce cas, le contact entre un réservoir donné et le suivant s'effectue après gonflement de ce réservoir donné. Les électrodes de détection sont par exemple disposées à l'interface entre les réservoirs ou au niveau de chaque réservoir.

L'invention peut être utilisée pour déterminer la quantité de sécrétion d'un liquide autre que la sueur, et peut s'étendre à tout autre liquide corporel (sang, exsudat, urine...).

## Revendications

1. Dispositif (10) de mesure de la quantité de sécrétion d'un liquide, comportant :
- une pluralité de réservoirs (11ᵢ) d'accumulation du liquide,
- une pluralité de structures de transfert (12ᵢ) éventuelles, les réservoirs étant reliés fluidiquement en série depuis une zone de captation dudit liquide, notamment par l'intermédiaire desdites structures de transfert,
- un système de détection (13ᵢ) de la présence du liquide au sein des réservoirs et/ou des structures de transfert, ce système de détection fournissant une information représentative de la quantité de liquide absorbée par le dispositif.

2. Dispositif selon la revendication 1, le système de détection opérant par mesure électrique, notamment par mesure de conductivité électrique.

3. Dispositif selon la revendication 1 ou 2, comportant les structures de transfert reliant fluidifiquement en série les réservoirs, les structures de transfert (12ᵢ) comportant de préférence des bandes drainantes.

4. Dispositif selon l'une quelconque des revendications 1 à 3, chaque réservoir (11ᵢ) comportant un espace apte à se combler progressivement sous l'effet de l'absorption de liquide dans le réservoir, le dispositif comportant une structure de transfert aval, espacée du réservoir correspondant avant absorption de liquide par celui-ci, le réservoir venant contacter cette structure de transfert aval lorsque ledit espace se comble.

5. Dispositif selon l'une quelconque des revendications précédentes, les réservoirs (11ᵢ) étant chacun en contact permanent avec une structure de transfert amont (12ⱼ₊₁) et une structure de transfert aval (12ⱼ), les réservoirs (11ᵢ) étant de préférence non superposés en vue de dessus.

6. Dispositif selon l'une quelconque des revendications 1 à 4, chaque réservoir (11ᵢ) comportant un matériau gonflant en présence dudit liquide, de manière à subir une expansion, le dispositif comportant une structure de transfert aval, espacée du réservoir correspondant avant expansion de celui-ci, le réservoir venant après son expansion contacter cette structure de transfert aval, les réservoirs (11ᵢ) étant de préférence superposés en vue de dessus.

7. Dispositif selon la revendication 6 , au moins une structure de transfert (12ᵢ) définissant une zone de transfert aval d'un réservoir, par laquelle le liquide quitte ce réservoir, et une zone de transfert amont du réservoir suivant, par laquelle le liquide arrive dans ce réservoir suivant.

8. Dispositif selon l'une quelconque des revendications précédentes, comportant une structure formant entretoise (17), maintenant espacées des zones de transfert amont et aval associées à un même réservoir.

9. Dispositif selon la revendication 7, les structures de transfert comportant une portion amont qui reçoit le fluide d'un réservoir et une portion aval qui transmet le fluide au réservoir suivant et une portion intermédiaire (23) qui relie les portions amont et aval et traverse la structure formant entretoise (17), le dispositif
comportant de préférence une électrode sur la portion intermédiaire (23).

10. Dispositif selon l'une quelconque des revendications précédentes, les réservoirs étant disposés chacun au moins partiellement au sein d'un logement réalisé dans une couche (30) d'un matériau hydrophobe.

11. Dispositif selon l'une quelconque des revendications 1 à 10, comportant des électrodes sur les structures de transfert (12ᵢ).

12. Dispositif selon l'une quelconque des revendications précédentes, comportant un moyen de fixation du dispositif sur la peau, notamment un bracelet (21).

13. Dispositif selon la revendication 1, les réservoirs étant superposés et constitués chacun d'un matériau gonflant sous l'effet de l'absorption du liquide, le système de détection comportant de préférence des électrodes qui détectent la présence de liquide à l'interface entre deux réservoirs ou au sein d'un réservoir, un réservoir étant isolé fluidifiquement du réservoir aval avant gonflement, et contactant ce réservoir aval après remplissage par le liquide et gonflement.

14. Dispositif selon l'une quelconque des revendications précédentes, le liquide étant un liquide physiologique.

15. Procédé de mesure de la quantité d'un liquide physiologique sécrété par une zone de peau, à l'aide d'un dispositif (10) selon l'une quelconque des revendications précédentes, comportant :
- la détection de la présence du liquide au niveau des structures de transfert (12ᵢ) et/ou des réservoirs (11ᵢ), et
la fourniture d'une information représentative de la quantité de liquide sécrétée, à partir desdites mesures

## Patentansprüche

1. Vorrichtung (10) zur Messung der Sekretionsmenge einer Flüssigkeit, mit:
- mehreren Behältern (11ᵢ), zum Sammeln der Flüssigkeit,
- mehreren eventuellen Transferstrukturen (12ᵢ), wobei die Behälter strömungsmäßig in Reihe mit einer Empfangszone für die genannte Flüssigkeit verbunden sind, insbesondere über die genannten Transferstrukturen,
- einem Detektionssystem (13ᵢ) zur Detektion der Anwesenheit der Flüssigkeit in den Behältern und/oder den Transferstrukturen, wobei das Detektionssystem eine Information liefert, die für die Menge der von der Vorrichtung aufgenommenen Flüssigkeit repräsentativ ist.

2. Vorrichtung nach Anspruch 1, bei der das Detektionssystem mit elektrischer Messung arbeitet, insbesondere Messung der elektrischen Leitfähigkeit.

3. Vorrichtung nach Anspruch 1 oder 2, mit Transferstrukturen, die die Behälter strömungsmäßig in Reihe verbinden, wobei die Transferstrukturen (12ᵢ) Drainagebänder aufweisen.

4. Vorrichtung nach einem der Ansprüche 1 bis 3, bei der jeder Behälter (11i) einen Raum aufweist, der in der Lage ist, sich unter der Wirkung der Aufnahme der Flüssigkeit in dem Behälter progressiv aufzufüllen, wobei die Vorrichtung eine stromabwärtige Transferstruktur aufweist, die vor der Aufnahme der Flüssigkeit in dem Behälter von diesem Behälter beabstandet ist, wobei der Behälter mit dieser stromabwärtigen Transferstruktur in Berührung kommt, wenn der genannte Raum sich füllt.

5. Vorrichtung nach einem der vorstehenden Ansprüche, bei der jeder der Behälter (11i) permanent mit einer stromaufwärtigen Transferstruktur (12ⱼ₊₁) und einer stromabwärtigen Transferstruktur (12ⱼ) in Kontakt steht, wobei die Behälter (11ᵢ) einander von oben gesehen vorzugsweise nicht überlappen.

6. Vorrichtung nach einem der Ansprüche 1 bis 4, bei der jeder Behälter (11ᵢ) ein sich in Gegenwart der genannten Flüssigkeit aufblähendes Material aufweist, derart, dass er eine Expansion erfährt, wobei die Vorrichtung eine stromabwärtige Transferstruktur aufweist, die vor der Expansion des Behälters von diesem Behälter beabstandet ist, und der Behälter nach seiner Expansion mit dieser stromabwärtigen Transferstruktur in Kontakt kommt und die Behälter (11ᵢ) von oben gesehen einander vorzugsweise überlappen.

7. Vorrichtung nach Anspruch 6, bei der wenigstens eine Transferstruktur (12ᵢ) eine stromabwärtige Transferstruktur eines Behälters bildet, durch welche die Flüssigkeit den Behälter verlässt, sowie eine stromaufwärtige Transferzone des nachfolgenden Behälters, über welche die Flüssigkeit in diesen nachfolgenden Behälter eintritt.

8. Vorrichtung nach einem der vorstehenden Ansprüche, mit einer Struktur, die einen Abstandshalter (17) bildet, der die stromaufwärtigen und stromabwärtigen Transferzonen desselben Behälters zueinander auf Abstand hält.

9. Vorrichtung nach Anspruch 7, bei der die Transferstrukturen einen stromaufwärtigen Abschnitt aufweisen, der das Fluid aus einem Behälter aufnimmt, und einen stromabwärtigen Abschnitt, der das Fluid an den nachfolgenden Behälter weiterleitet, sowie einen mittleren Abschnitt (23), der die stromaufwärtigen und stromabwärtigen Abschnitte verbindet und die den Abstandshalter (17) bildende Struktur durchquert, wobei die Vorrichtung
vorzugsweise eine Elektrode auf dem mittleren Abschnitt (23) aufweist.

10. Vorrichtung nach einem der vorstehenden Ansprüche, bei der die Behälter jeweils zumindest zum Teil in einer Aufnahme angeordnet sind, die in einer Schicht (30) aus einem hydrophoben Material gebildet ist.

11. Vorrichtung nach einem der Ansprüche 1 bis 10, mit Elektroden auf den Transferstrukturen (12ᵢ).

12. Vorrichtung nach einem der vorstehenden Ansprüche, mit einem Mittel zur Befestigung der Vorrichtung auf der Haut, insbesondere einem Armband.

13. Vorrichtung nach Anspruch 1, bei der die Behälter einander überlagert sind und jeweils aus einem Material gebildet sind, das sich unter der Wirkung der Aufnahme der Flüssigkeit aufbläht, wobei das Detektionssystem vorzugsweise Elektroden aufweist, die die Anwesenheit der Flüssigkeit an der Schnittstelle zwischen zwei Behältern oder im Inneren eines Behälters detektieren, wobei ein Behälter vor dem Aufblähen fluidmäßig von dem stromabwärtigen Behälter isoliert ist und mit diesem stromabwärtigen Behälter nach dem Auffüllen mit der Flüssigkeit und dem Aufblähen in Berührung steht.

14. Vorrichtung nach einem der vorstehenden Ansprüche, bei der die Flüssigkeit eine physiologische Flüssigkeit ist.

15. Verfahren zur Messung der Menge einer physiologischen Flüssigkeit, die von einem Hautbereich sekretiert wurde, mit Hilfe einer Vorrichtung (10) nach einem der vorstehenden Ansprüche, umfassend:
- die Detektion der Anwesenheit der Flüssigkeit an der Stelle der Transferstrukturen (12ᵢ) und/oder der Behälter (11ᵢ) und
Liefern einer Information, die für die Menge an sekretierter Flüssigkeit repräsentativ ist, anhand der genannten Messungen.

## Claims

1. Device (10) for measuring the amount of secretion of a liquid, comprising:
- a plurality of containers (11ᵢ) for accumulating the liquid,
- a plurality of possible transfer structures (12ᵢ), the containers being connected fluidly in series from an area for capturing this liquid, particularly through these transfer structures,
- a system for detecting (13ᵢ) the presence of the liquid within the containers and/or transfer structures, this detection system providing information representing the amount of liquid absorbed by the device.

2. Device according to claim 1, the detection system operating by electrical measurement, particularly by measuring electrical conductivity.

3. Device according to claim 1 or 2, comprising the transfer structures connecting the containers fluidly in series, the transfer structures (12ᵢ) preferably comprising draining bands.

4. Device according to any one of claims 1 to 3, each container (11ᵢ) comprising a space that is able to be filled progressively under the effect of the absorption of liquid in the container, the device comprising a downstream transfer structure, which is at a distance from the corresponding container before absorption of liquid by it, the container coming into contact with this downstream transfer structure when this space is filled.

5. Device according to any one of the previous claims, the containers (11ᵢ) each being in permanent contact with an upstream transfer structure (12ⱼ₊₁) and a downstream transfer structure (12ⱼ), the containers (11ᵢ) preferably not being superimposed when seen from above.

6. Device according to any one of claims 1 to 4, each container (11ᵢ) comprising a material that swells in the presence of this liquid so as to undergo an expansion, the device comprising a downstream transfer structure, which is at a distance from the corresponding container before its expansion, the container coming into contact with this downstream transfer structure after its expansion, the containers (11ᵢ) preferably being superimposed when seen from above.

7. Device according to claim 6, at least one transfer structure (12ᵢ) defining a downstream transfer area of a container, through which the liquid leaves this container, and an upstream transfer area of the following container, through which the liquid arrives in this following container.

8. Device according to any one of the previous claims, comprising a structure forming a spacer (17), keeping the upstream and downstream transfer areas associated with one and the same container at a distance.

9. Device according to claim 7, the transfer structures comprising an upstream portion, which receives the fluid from a container, and a downstream portion, which transfers the fluid to the following container, and an intermediate portion (23), which connects the upstream and downstream portions and crosses the structure forming a spacer (17), the device preferably comprising an electrode on the intermediate portion (23).

10. Device according to any one of the previous claims, the containers each being arranged at least partially within a housing made in a layer (30) of a hydrophobic material.

11. Device according to any one of claims 1 to 10, comprising electrodes on the transfer structures (12ᵢ).

12. Device according to any one of the previous claims, comprising a means of fixing the device on the skin, particularly a bracelet (21).

13. Device according to claim 1, the containers being superimposed and each made of a material that swells under the effect of absorption of the liquid, the detection system preferably comprising electrodes, which detect the presence of liquid at the interface between two containers or within a container, a container being isolated fluidly from the downstream container before swelling and coming into contact with this downstream container after filling by the liquid and swelling.

14. Device according to any one of the previous claims, the liquid being a physiological liquid.

15. Method for measuring the amount of a physiological liquid secreted by an area of skin, using a device (10) according to any one of the previous claims, comprising:
- detection of the presence of the liquid at the level of the transfer structures (12ᵢ) and/or the containers (11ᵢ) and providing information representing the amount of liquid secreted from these measurements
